(19) Europäisches Patentamt
European Patent Office
Office européen des brevets

(11) **EP 4 203 876 B1**

(12) **EUROPEAN PATENT SPECIFICATION**

(45) Date of publication and mention
of the grant of the patent:
**29.04.2026   Bulletin 2026/18**

(21) Application number: **21773420.1**

(22) Date of filing: **26.08.2021**

(51) International Patent Classification (IPC):
**A61F 9/008** *(2006.01)*      **A61F 9/009** *(2006.01)*

(52) Cooperative Patent Classification (CPC):
**A61F 9/00831;** A61F 9/009; A61F 2009/00872;
A61F 2009/00882

(86) International application number:
**PCT/IB2021/057842**

(87) International publication number:
**WO 2022/043921 (03.03.2022 Gazette 2022/09)**

(54) **POSTERIOR CORNEAL SURFACE MAPPING AND DEEP LAMELLAR CORNEAL INCISION PARALLEL TO POSTERIOR CORNEAL SURFACE**

POSTERIORE HORNHAUTOBERFLÄCHENKARTIERUNG UND TIEFE LAMELLARE HORNHAUTINZISION PARALLEL ZUR HINTEREN HORNHAUTOBERFLÄCHE

CARTOGRAPHIE DE LA SURFACE CORNÉENNE POSTÉRIEURE ET INCISION LAMELLAIRE CORNÉENNE PROFONDE PARALLÈLE À LA SURFACE CORNÉENNE POSTÉRIEURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2020   US 202063072066 P**

(43) Date of publication of application:
**05.07.2023   Bulletin 2023/27**

(73) Proprietor: **AMO Development, LLC**
**Irvine, CA 92618 (US)**

(72) Inventors:
• **FU, Hong**
**Irvine, California 92618 (US)**
• **RAHAMAN, Mohammad Saidur**
**Irvine, California 92618 (US)**
• **MALEK TABRIZI, Alireza**
**Irvine, California 92618 (US)**
• **WITOWSKI, Zenon**
**Pleasanton, California 94566 (US)**
• **ALTMANN, Griffith**
**Ladera Ranch, California 92694 (US)**

(74) Representative: **Carpmaels & Ransford LLP**
**One Southampton Row**
**London WC1B 5HA (GB)**

(56) References cited:
**US-A1- 2015 272 782      US-A1- 2015 374 549**
**US-A1- 2016 374 857      US-A1- 2020 064 622**

## Description

BACKGROUND OF THE INVENTION

Field of the Invention

This invention relates to forming corneal incisions using ultra-short pulsed laser, and in particular, it relates to forming deep lamellar incisions parallel to the posterior corneal surface and a posterior corneal surface mapping method.

Description of Related Art

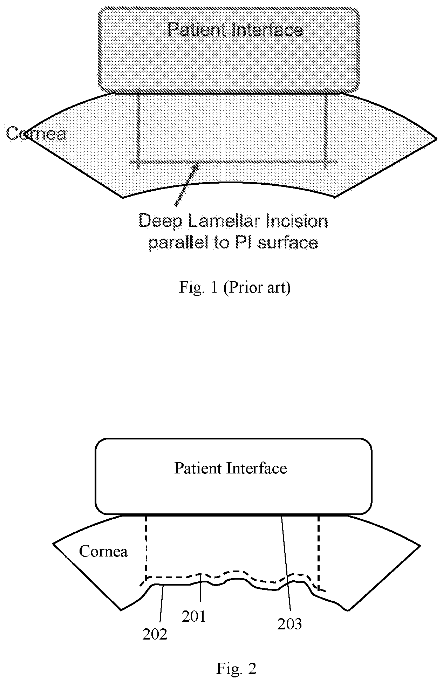

Deep anterior lamellar keratoplasty (DALK) is a corneal transplant technique. It cuts and removes an anterior corneal button from the recipient eye, then replaces it with an anterior corneal button cut from a donor cornea. Ultra-short pulsed laser systems, such as femtosecond lasers, has been applied to this procedure. In conventional DALK techniques, the deep lamellar incision profile, for example, a planar dissection, is typically set to be parallel to the surface of the patient interface device (a device used to engage and restrain the patient's eye during ophthalmic procedures), as schematically illustrated in Fig. 1. Due to the lack of posterior corneal surface mapping, a surgeon usually leaves sufficient margin between the incision and the posterior corneal surface, and the remaining thin tissue bed may have uneven thickness due to possible posterior corneal folding when the eye is engaged with the patient interface device.

Methods have also been described which set the corneal dissection profile in parallel to the posterior corneal surface when cutting a corneal button, for example, in U.S. Pat. Appl. Pub. No. 20070027438, entitled "System and method for compensating a corneal dissection," and U.S. Pat. No. 10080684, entitled "System and method for laser corneal incisions for keratoplasty procedures." These references describe measuring the topology of the cornea, including the posterior corneal surface, using methods such as wavefront analysis of the reflected diagnostic beam, ellipsometry, second harmonic generation (SHG) microscopy, confocal microscopy, corneal topography, optical coherence tomography (OCT), or ultrasonic pachymetry, Purkinje imaging, Scheimpflug imaging, confocal or nonlinear optical microscopy, fluorescence imaging, ultrasound, structured light, or stereo imaging (see US 20070027438 , [0034], and US 10080684, col. 17, lines 14-28). In these references, the anterior cornea surface of the eye is not shown as being applanated by the surface of the patient interface device (see, e.g., Fig. 4B of US 20070027438 and Fig. 5A of US 10080684).

US 2015/0374549 AI provides systems and methods for laser corneal incisions for keratoplasty procedures. A first image of the eye is generated when the cornea of the eye is exposed to a gas. The cornea is covered with an optic of a patient interface. A second image of the eye with the patient interface over the cornea is generated. In this second image, the patient interface distorts the second image of the eye. One or more of a position or an orientation of the eye is determined in response to the first image and the second image when the patient interface has been placed over the cornea.

US 2015/0272782 AI provides a laser surgery system that includes a light source, an eye interface device, a scanning assembly, a confocal detection assembly and preferably a confocal bypass assembly. The light source generates an electromagnetic beam. The scanning assembly scans a focal point of the electromagnetic beam to different locations within the eye. An optical path propagates the electromagnetic beam from a light source to the focal point, and also propagates a portion of the electromagnetic beam reflected from the focal point location back along at least a portion of the optical path. The optical path includes an optical element associated with a confocal detection assembly that diverts a portion of the reflected electromagnetic radiation to a sensor. The sensor generates an intensity signal indicative of intensity the electromagnetic beam reflected from the focal point location. US2020/06422A1 provides systems and methods for detection of an optical surface of a patient interface for ophthalmic laser applications using a non-confocal configuration.

SUMMARY

The scope of the invention is defined by the claims which follow. Methods described are given for illustrative purposes only. Embodiments of the present invention are directed to a system configured for forming deep corneal lamellar incision that is parallel to the posterior corneal surface, in which a lower-energy detecting beam generated by the same pulsed laser that generates the higher-energy treatment laser beam is utilized to measure the posterior corneal surface profile when the eye is docked to the patient interface.

Additional features and advantages of the invention will be set forth in the descriptions that follow and in part will be apparent from the description, or may be learned by practice of the invention. The objectives and other advantages of the invention will be realized and attained by the structure particularly pointed out in the written description and claims thereof as well as the appended drawings.

To achieve the above objects, the present invention provides an ophthalmic laser system that includes: a pulsed laser configured to generate a pulsed laser beam; an optical system including an objective lens and one or more scanners, configured to focus the laser beam to a focus point and to scan the focus point in three directions in an eye of a patient; a light intensity detector disposed to detect a light intensity of a back-reflected laser beam from the eye that has passed through the objective lens; a controller electrically coupled to the pulsed laser, the optical system and the light intensity detector, wherein the controller is configured to: control the pulsed laser to generate a first laser beam having a first pulse energy lower than a threshold energy for photodisruption of corneal tissue; control the optical system to scan the focus point of the first laser beam according to a first scan pattern, wherein the first scan pattern is at least partially located within a cornea of the eye and extends in a predetermined depth range in a depth direction which is parallel to an optical axis of the objective lens; control the detector to detect a light intensity signal of a back-reflected portion of the first laser beam while the focus point of the first laser beam is scanned according to the first scan pattern; based on a correspondence between the light intensity signal and the first scan pattern, determine a depth profile of a posterior corneal surface of the eye; control the pulsed laser to generate a second laser beam having a second pulse energy higher than the threshold energy; and based on the determined depth profile of the posterior corneal surface, control the optical system to scan the focus point of the second laser beam within the cornea according to a second scan pattern, the second scan pattern having a defined spatial relationship with the determined posterior corneal surface profile, to form an incision in the cornea.

In some embodiments, the one or more scanners includes a Z scanner and an X-Y scanner, and the controller is configured to scan the focus point of the first laser beam according to the first scan pattern by: controlling the Z scanner to scan the focus point in the depth direction according to an oscillating function of time; and simultaneously controlling the X-Y scanner to scan the focus point in a spiral pattern in a plane perpendicular to the depth direction.

In some embodiments, the one or more scanners includes a Z scanner, an X-Y scanner, and a resonance scanner, and the controller is configured to scan the focus point of the first laser beam according to the first scan pattern by: controlling the Z scanner to scan the focus point in the depth direction according to a oscillating function of time; simultaneously controlling the X-Y scanner to scan the focus point in a spiral pattern in a plane perpendicular to the depth direction; and simultaneously controlling the resonant scanner to scan the focus point into a scanline in the plane at a frequency higher than a frequency of the oscillating function of the Z scanner.

In some embodiments, the one or more scanners includes a Z scanner, an X-Y scanner, and a resonance scanner, and the controller is configured to scan the focus point of the first laser beam according to the first scan pattern by: controlling the Z scanner and the X-Y scanner to scan the focus point in three dimensions according to a pattern; and simultaneously controlling the resonant scanner to scan the focus point into a scanline in a plane perpendicular to the depth direction.

Here described is also an ophthalmic laser surgery method implemented in an ophthalmic laser system, which includes: coupling an eye of a patient to a patient interface device of the ophthalmic laser system; while the eye is coupled to the patient interface: by a laser source of the ophthalmic laser system, generating a first laser beam having a first pulse energy lower than a threshold energy for photodisruption of corneal tissue; by an objective lens, focusing the first laser beam to a focus point; by one or more scanners of the ophthalmic laser system, scanning the focus point of the first laser beam according to a first scan pattern, wherein the first scan pattern is at least partially located within a cornea of the eye and extends in a predetermined depth range in a depth direction which is parallel to an optical axis of the objective lens; by a light intensity detector of the ophthalmic laser system, detecting a light intensity signal of a back-reflected portion of the first laser beam while the focus point of the first laser beam is scanned according to the first scan pattern; by a controller of the ophthalmic laser system, based on a correspondence between the light intensity signal and the first scan pattern, determining a depth profile of a posterior corneal surface of the eye; by the pulsed laser, generating a second laser beam having a second pulse energy higher than the threshold energy; by the objective lens, focusing the second laser beam to a focus point; and by the one or more scanners, based on the determined depth profile of the posterior corneal surface, scanning the focus point of the second laser beam within the cornea according to a second scan pattern, the second scan pattern having a defined spatial relationship with the determined posterior corneal surface profile, to form an incision in the cornea.

In some embodiments, the step of scanning the focus point of the first laser beam according to the first scan pattern includes: by a Z scanner of the ophthalmic laser system, scanning the focus point in the depth direction according to an oscillating function of time; and simultaneously, by an X-Y scanner of the ophthalmic laser system, scanning the focus point in a spiral pattern in a plane perpendicular to the depth direction

In some embodiments, the step of scanning the focus point of the first laser beam according to the first scan pattern includes: by a Z scanner of the ophthalmic laser system, scanning the focus point in the depth direction according to an oscillating function of time; simultaneously, by an X-Y scanner of the ophthalmic laser system, scanning the focus point in a spiral pattern in a plane perpendicular to the depth direction; and simultaneously, by a resonant scanner of the ophthalmic laser system, scanning the focus point into a scanline in the plane at a frequency higher than a frequency of the oscillating function of the Z scanner.

In some embodiments, the step of scanning the focus point of the first laser beam according to the first scan pattern includes: by a Z scanner and an X-Y scanner of the ophthalmic laser system, scanning the focus point in three dimensions according to a pattern; and simultaneously, by a resonant scanner of the ophthalmic laser system, scanning the focus point into a scanline in a plane perpendicular to the depth direction.

It is to be understood that both the foregoing general description and the following detailed description are exemplary and explanatory and are intended to provide further explanation of the invention as claimed.

BRIEF DESCRIPTION OF THE DRAWINGS

Figure 1 schematically illustrates a deep lamellar corneal incision made parallel to the surface of the patient interface (PI) that is engaged with the eye, according to conventional technologies.

Figure 2 schematically illustrates a deep lamellar corneal incision made parallel to the posterior cornea surface when the eye is docked to a patient interface, according to embodiments of the present invention.

Figure 3 schematically illustrates a portion of an ophthalmic laser system that uses a non-confocal detection configuration for measuring the Z position of the posterior corneal surface or other reflective interfaces according to an embodiment of the present invention.

Figures 4A and 4B schematically illustrate the principle of measuring the Z position of a reflective interface in the laser system of Fig. 3.

Figure 5 is a flow chart of a method of forming a deep lamellar corneal incision parallel to posterior corneal surface.

Figure 6 is a flow chart of a method of measuring a 3-dimensional profile of the posterior corneal surface.

Figure 7 schematically illustrates a detecting beam being focused inside the cornea during the measurement process of Fig. 6.

Figure 8 shows an example of the auto-Z signal obtained from a depth measurement using the method of Fig. 6.

Figure 9 is a flow chart of another method of measuring a 3-dimensional profile of the posterior corneal surface.

Figure 10A schematically illustrates a spiral pattern of an X-Y scan employed in the method of Fig. 8.

Figure 10B schematically illustrates a sinusoidal fast-Z scan pattern employed in the method of Fig. 8.

Figures 11A and 11B schematically illustrate two exemplary ophthalmic surgical laser systems in which the described methods may be implemented.

DETAILED DESCRIPTION OF PREFERRED EMBODIMENTS

Here described is a method that utilizes a detecting laser beam, generated by the same pulsed laser that generates the higher-energy treatment laser beam, to measure the posterior corneal surface profile, and to form a deep corneal lamellar incision parallel to the measured posterior corneal surface. Because a beam from the same pulsed laser that generates the treatment beam is used to measure the posterior corneal surface profile, calibration error is significantly reduced or eliminated. This is more advantageous than using the optical coherence tomography (OCT) technique to measure the posterior corneal surface, as OCT uses a light beam not generated by the treatment laser. The method described here has a precision of approximately 1 $\mu$m, which is higher than that of the OCT technique (approximately 10 $\mu$m). This higher precision is advantageous for performing deep incisions close to the posterior corneal surface.

Fig. 2 schematically illustrates a deep lamellar corneal incision 201 made parallel to the posterior cornea surface 202 of an eye docked to a patient interface, according to embodiments of the present invention. As shown in Fig. 2, during the procedure, the eye is docked to the patient interface, where the anterior corneal surface 203 is flattened by the surface of the patient interface. As schematically shown in Fig. 2, the posterior corneal surface 202 may have a somewhat flattened shape, with folds and other irregular shapes, which are to be measured by the anterior corneal surface mapping method described herein.

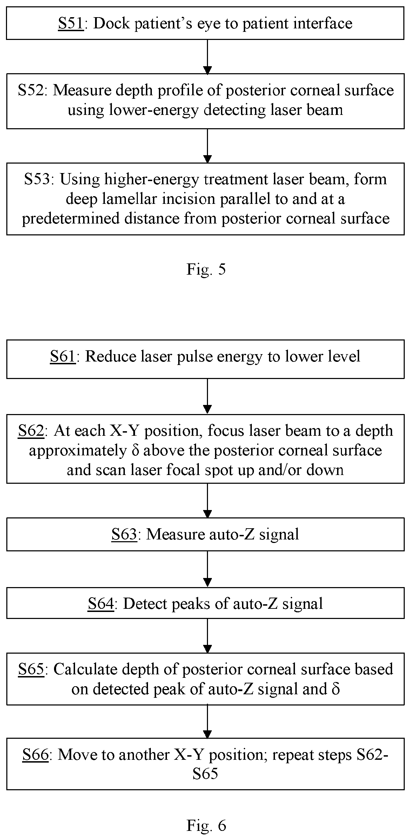

To form a deep lamellar corneal incision parallel to posterior corneal surface, first , the patient's eye is docked to the patient interface device (Fig. 5, step S51). The depth profile of the posterior corneal surface is measured using a lower-energy detecting laser beam generated by the treatment laser, using a method described in more detail below (step S52). The depth profile (or simply the profile) of the posterior corneal surface describes the depth value (Z, in the direction parallel to the optical axis of the laser system) of the surface as a function of the position (X-Y) in a plane perpendicular to the optical axis. Then, based on the measured posterior corneal surface profile, and using the higher-energy treatment laser beam, a deep lamellar incision is formed parallel to and at a predetermined distance from the posterior corneal surface (step S53). Step S53 includes planning a treatment scan pattern based on the measured posterior corneal surface profile, and scanning the treatment laser beam according to the treatment scan pattern. Steps S52 and S53 are performed while the eye is continuously docked to the patient interface device. The detecting laser beam and the treatment laser beam are generated by the same laser source.

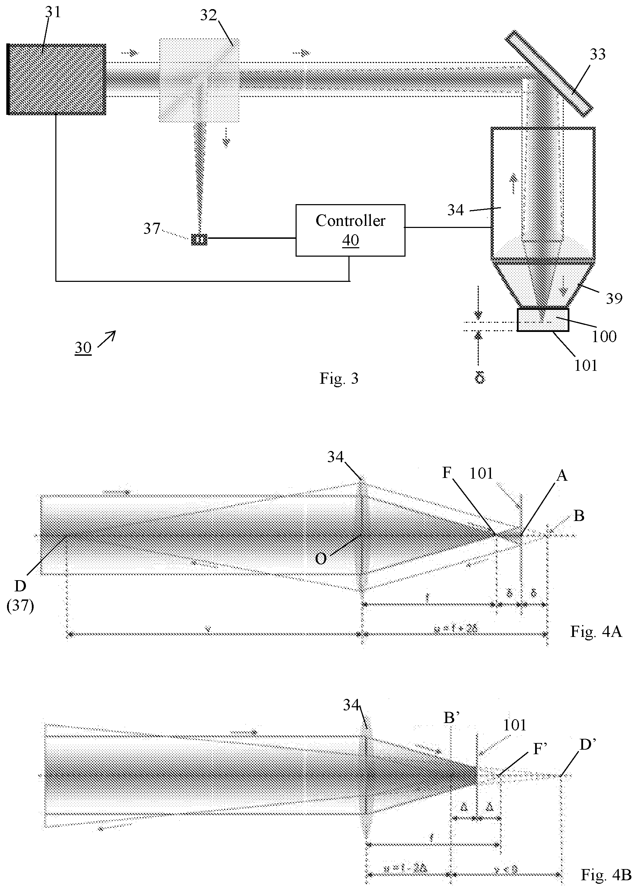

The principles of depth measurement is described below with reference to Figs. 3, 4A and 4B.

Fig. 3 schematically illustrates a portion of an ophthalmic laser system 30 that uses a non-confocal detection configuration for measuring the depth (Z) position of a reflective interface according to an embodiment of the present invention. In the system shown in Fig. 3, the laser source 31, the details of which are not shown, includes a laser device and associated optical components, including an X-Y scanner, that are configured to produce a laser beam and scan the beam in an X-Y plane perpendicular to the optical axis. A part of the laser beam passes through a beam splitter 32, and after being reflected by one or more mirrors 33 (optional), is focused by the objective lens 34. In a preferred embodiment, the objective lens 34 has a relatively high numerical aperture (NA), for example, approximately 0.4 or higher. The focus spot size produced by the objective lens is preferably as small as 2 $\mu$m, or 1 $\mu$m, or even smaller.

The objective lens 34 is mounted on a movement structure and moveable in the Z direction (parallel to the optical axis) relative to a housing of the laser system, so as to focus the laser beam at desired depths and to vary the depth of the focus spot. The movement structure may include any suitable mechanical structure, such as a translation stage driven by a motor, etc.

A part of the laser light that exits the objective lens 34 is reflected by a reflective (including partially reflective) interface 101 located below the objective lens 34, and the reflected light travels backwards into the objective lens 34. After the back-reflected light is focused by the objective lens 34 and reflected by the mirror 33, a part of the reflected light is reflected by the beam splitter 32 onto a small two-dimensional light intensity detector 37 (e.g. a photodetector). In the illustrated embodiment, no confocal lens or pinhole is used in front of the detector 37.

The reflective interface 101 is an interface whose depth is being measured. In this embodiment, the reflective interface is the posterior corneal surface, but it can be any reflective interface, including, for example, a surface of contact lens of the patient interface, and the depth measurement principle described below applies equally. In Fig. 3, the patient interface is schematic illustrated as component 39, and the cornea is schematically illustrated as component 100 with the posterior surface 101 being measured. Alternatively, when the interface being measured is a distal surface of the patient interface lens, then component 100 may represent the patient interface lens with a distal surface 101, and component 39 may represent a housing of the patient interface.

A controller 40 controls the operations of the laser source 31, objective lens 34, and detector 37. The controller may be implemented by electrical circuitry including logic circuits, and/or processors which execute computer executable program code stored in computer readable non-volatile memories.

The principle of Z position measurement using the laser system of Fig. 3 is described below with reference to the schematic illustrations in Figs. 4A and 4B. In Figs. 4A and 4B, the objective lens 34 is optically represented by a thin lens having a focal distance f, although the objective lens is typically formed of a set of lenses. It should be noted that Figs. 4A and 4B are intended to explain the relevant optical principles; the various distances depicted in the figures are not to scale.

Fig. 4A illustrates a situation where the objective lens 34 focuses the parallel incident beam to a focus point F located between the objective lens 34 and the reflective interface 101. In other words, the reflective interface 101 is located beyond the focal plane of the objective lens 34. The forward propagating light diverges after the focus point F and is then reflected by the reflective interface 101. To the objective lens 34, the reflected light appears to originate from a point B behind the reflective interface 101, the point B being the mirror image of the focus point F with respect to the reflective interface 101. The distance from the equivalent origin B to the objective lens 34 is $u = f + 2\delta$ (Equation (1)), where $\delta$=FA is the offset distance between the focus point F and the reflective interface 101. The reflected light from the equivalent origin B is focused by the objective lens 34 to an image point D at a finite distance v from the objective lens. The detector 37 is located at the image point D, and no other lens is disposed between the objective lens 34 and the detector 37.

Using Equation (1) and the following lens formula for a thin lens (Equation (2)),

$$\frac{1}{u} + \frac{1}{v} = \frac{1}{f},$$

where u is the object distance and v is the image distance, one obtains (Equation (3)):

$$\delta = \frac{f^2}{2(v-f)}$$

When f is much smaller than v (discussed later), one obtains (Equation (4)):

$$\delta \approx \frac{f^2}{2v}$$

The above equations are for focusing in the air. When the focus point F is located inside an optical medium (e.g. the cornea), the refractive index n of the optical medium is taken into consideration, and one obtains (Equation (5)) (note here that the refractive index n is an effective index of the optical system which has multiple components and different materials):

$$\delta \approx n \cdot \frac{f^2}{2v}$$

It should be understood that in the above equations, the various distances are the distances along the optical path; the optical path may be folded by mirrors or beam splitters.

In the laser system 30, the distance DO from the detector 37 to the objective lens 34 (i.e. the imaging distance v) is approximately a device constant, because the location of the detector 37 is fixed relative to the laser system housing and the amount of focusing movement of the objective lens 34 with respect to the housing is much smaller than the distance DO. Therefore, the distance $\delta$ given by Equation (5) is approximately a constant of the laser system 30. The point located at distance $\delta$ before the reflective interface 101 is referred to as the target focus position for convenience. If the light is focused by the objective lens 34 at this target focus position, the reflected light from the reflective interface 101 will be focused onto the detector 37.

In some embodiments, the focal distance *f*, i.e. the equivalent focal length of the objective lens 34, is a few mm, e.g. approximately 4 mm. Meanwhile, the distance DO from the detector 37 to the objective lens 34 may be several hundreds of mm (e.g. 500 mm), because the choice of the detector location is not constrained and the image distance v may be lengthened if desired by folding the optical path with mirrors. Therefore, *f* is much smaller than v (by a factor on the order of 100). In one particular embodiment, where the image distance v (DO) is approximately 500 mm and the equivalent focal length *f* is 3.92 mm, using the refractive index of the optical medium (cornea) of n=1.38, Equation (5) gives $\delta \approx 21\ \mu m$.

When the objective lens 34 focuses the laser beam at positions other than the target focus position defined by $\delta$, the back-reflected light will not be focused on the detector 37 located at point D, but will be focused before it, after it, or not be focused at all. Fig. 4B schematically illustrates an example where the focus point F' of the objective lens 34 is located beyond the reflective interface 101. The light from the objective lens 34 converges as it strikes the reflected interface 101 and is reflected by it; therefore, to the objective lens 34, the back-reflected light appears to originate from a point B' before the reflective interface 101, the point B' being the mirror image of the focus point F' with respect to the reflective interface 101. Since the distance from the point B' to the objective lens 34 is shorter than the focal distance, the back-reflected light remains divergent after it passes through the lens 34. As illustrated in Fig. 4B, the distance from the focus point F' to the reflective interface 101 is denoted $\Delta$; the back-reflected light has an object distance $u = f - 2\Delta$, and forms a virtual image at a point D' behind the lens 34.

To summarize, the back-reflected light from the reflective interface 101 will only form a well focused real image on the detector 37 when the objective lens 34 focuses the parallel beam to the target focus position defined by $\delta$ (Equation (5)). The detector 37 has a relatively small effective detection area, such as about 1 mm$^2$ or smaller. Having a small effective detection area refers to the detector either having a physically small detection area or being controlled to detect light falling within a small area. As a result, when the back-reflected light is not focused on the detector, substantial portions of the reflected light will not fall on the effective detection area and the detected light intensity will decrease significantly. Therefore, the light intensity signal detected at the detector 37 (referred to as the auto-Z signal) peaks when the objective lens focuses the parallel laser beam to the target focus position. Thus, by continuously moving the objective lens 34 in the Z direction and continuously detecting the auto-Z signal, the target focus position can be measured (e.g., as expressed by the corresponding position of the objective lens); from the target focus position, the Z position of the reflective interface 101 can be obtained using the value of $\delta$.

A calibration process may be carried out by using the above technique to measure the Z position of a reference surface, for example, the distal surface of the patient interface lens. In the calibration process, the laser beam is focused at a distance $\delta$ before the reference which acts as the reflective interface 101. After such calibration, the Z position of the posterior corneal surface may be expressed as a distance relative to the reference surface.

The technique described above can achieve a 1 $\mu m$ or higher depth resolution of the Z position detection.

The above depth measurement technique is also described in commonly owned, copending application U.S. Pat. Appl. No. 16/112507, filed Aug. 24, 2018, entitled "Detection of optical surface of patient interface for ophthalmic laser applications using a non-confocal configuration,". US 16/112507 also describes a method of empirically measuring the value of the offset distance $\delta$ for a given laser system, without using Equation (5).

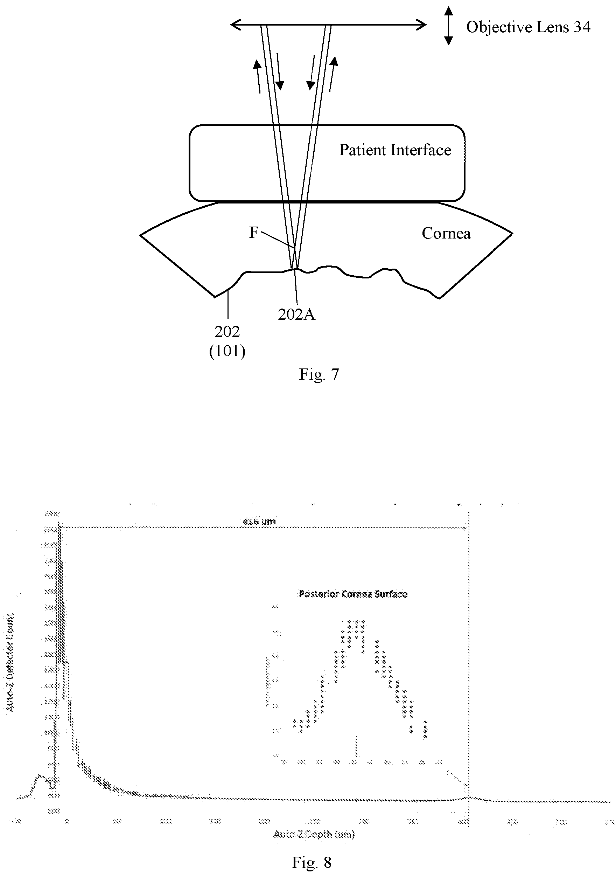

Referring to Figs. 6 and 7, using the above-described technique, the step of measuring the depth profile of the posterior corneal surface (step S52) may be performed as follows. The laser pulse energy is reduced to an appropriate lower level below a threshold energy that causes photodisruption of corneal tissue (step S61). The lower pulse energy level may be, for example, approximately 1 nJ per pulse (which is more than 30 times below the photodisruption threshold), or more generally, between 1 nJ and 10 nJ. The objective lens (or another Z scanner) is operated to focus the detecting laser beam to a focal spot (focus point) F located above the posterior corneal surface, starting at an initial distance approximately (may be greater or less than) $\delta$ above the posterior corneal surface. The objective lens (or the other Z scanner) is operated scan the focal spot upwardly and/or downwardly (step S62), while the auto-Z signal at the detector 37 is measured (step S63). The peak(s) of the auto-Z signal is(are) determined (step S64). The depth of the posterior corneal surface is calculated from the focal spot depths corresponding to the peaks of the auto-Z signal, by adding the known offset distance $\delta$ to the focal spot depths (step S65). Steps S62 to S65 are performed at each X-Y position, and repeated for different X-Y positions to obtain a 3-dimensional profile of the posterior corneal surface (step S66). The X-Y position of the focal spot is controlled by the X-Y scanner of the laser source 31. In this process, the controller 40 controls and synchronizes the operations of the X-Y and Z scanner and the detector and performs the relevant data processing functions.

Referring to Fig. 7, the size of an area 202A of the posterior corneal surface 202 that is illuminated by the light cone of the detecting beam depends on the numerical aperture of the objective lens and the distance from the focal spot F to the posterior corneal surface. In preferred embodiments, when the focal spot is at distance $\delta$ from the posterior corneal surface, the diameter of the illuminated area is approximately 10 to 20 $\mu$m, for example, approximately 17 $\mu$m in one particular example. Therefore, the depth measured by the auto-Z signal is an average of the illuminated area 202A; this reduces the impact of depth fluctuations on the posterior corneal surface. Note that the sizes of the folds in the posterior corneal surface, which are the object of the depth measurement, are much larger than the size of the illuminated area 202A.

Fig. 8 shows an exemplary auto-Z signal detected using the method of Fig. 6 with a rabbit cornea, for a given X-Y position. Note that the high peak at depth $\approx 0$ $\mu$m corresponds to the anterior corneal surface, i.e. the interface between the cornea and the patient interface lens. This example shows that the auto-Z signal corresponding to the posterior corneal surface, although much lower that the signal corresponding to the anterior corneal surface, has sufficient signal-to-noise ratio to allow for an accurate detection of the posterior corneal surface. Using the reflectivity formula for an optical interface, $R = (n1-n2)^2/(N1+N2)^2$, it can be estimated that the reflectivity is about 3.5% at the interface of the patient interface lens (glass) and air, about 0.07% at the anterior corneal surface (glass-cornea interface), and about 0.03% at the posterior corneal surface (cornea-water interface). The above example shows that the depth measurement method described here can measure all these reflections with sufficient signal-to-noise ratio. This example also shows that the precision that one can resolve the location of the signal peak is 1-2 $\mu$m, or about 1 $\mu$m.

In an alternative example of the depth measurement method, step S62 is modified as follows. For each X-Y position, at a given depth, the laser focal spot is scanned, preferably at high speed, over a small area in the X-Y plane centered at the given X-Y position. The auto-Z signal measured during such X-Y scan (in step S63), at the given depth, is an integrated signal from the scanned area . The depth measured by such integrated auto-Z signal represents the average depth of the corneal posterior surface corresponding to the scanned area. A resonant scanner, described in more detail later, may be used to perform such X-Y scan by scanning the focal spot into a short scanline of, for example, about 100 $\mu$m long (more generally, between 50 and 200 $\mu$m). This alternative method reduces laser exposure at a given tissue location, because the laser spot is spread out while the auto-Z signal is integrated.

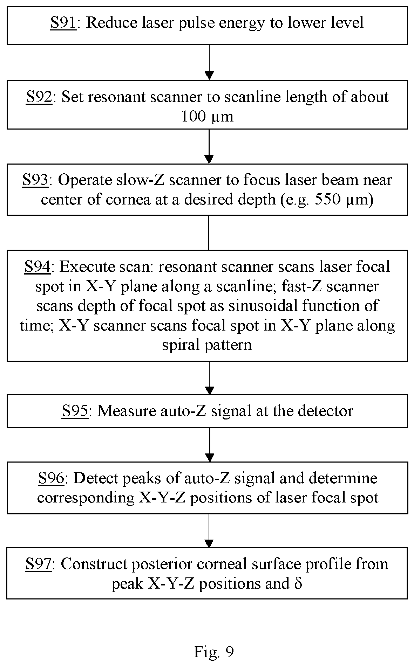

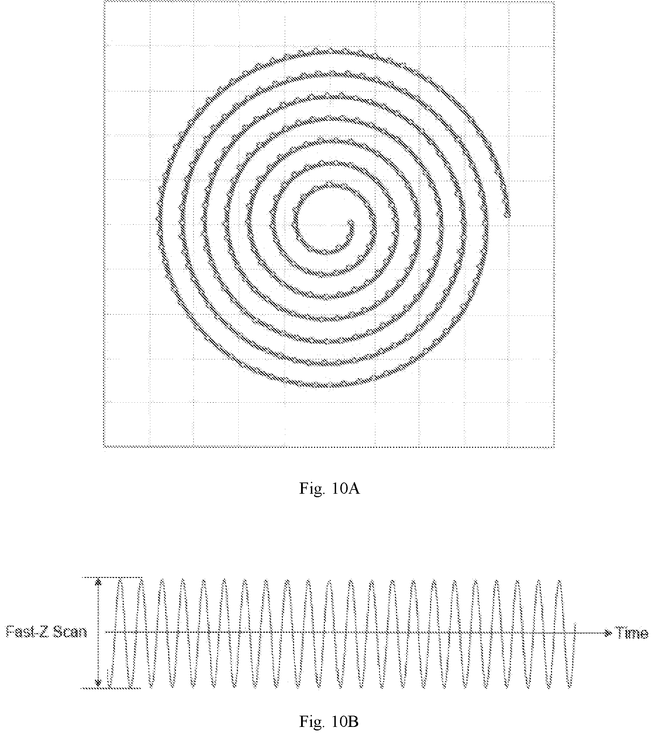

Another example of the method for obtaining a posterior corneal surface profile (step S52 of Fig. 5) is described with reference to Figs. 9, 10A and 10B. As shown in Fig. 9, first, the laser pulse energy is reduced to an appropriate lower level (step S91), for example, approximately 1 nJ per pulse. The resonant scanner is set to scan a scanline length of about 100 $\mu$m (step S92). A slow-Z scanner (e.g., the objective lens 34) is operated to focus the detecting laser beam near the center of the cornea at an initial depth below the anterior corneal surface (the interface between the cornea and the patient interface lens), such as approximately 550 $\mu$m, which is close to but not necessarily equal to the central corneal thickness (step S93). A typical thickness of the human cornea is about 540-560 $\mu$m.

A three-dimensional scan is then executed using the resonant scanner, a fast-Z scanner, and the X-Y scanner of the laser source 31 operating simultaneously (step S94). The fast-Z scanner scans the depth of the focal spot as a sinusoidal function (or other oscillating function) of time at an amplitude of about 100 $\mu$m (or more generally, at an amplitude between 10 um and 100 $\mu$m) (see Fig. 10B). At the same time, the X-Y scanner scans the focal spot in the X-Y plane along a spiral pattern (see Fig. 10A). Meanwhile (optional), the resonant scanner scans the focal spot at a relatively high frequency along a short scanline in the X-Y plane. The scanlines may be in the same X-Y direction throughout, or they may be rotated by a scanline rotator. The frequency of the resonant scanner is typically much higher than the frequency of the fast-Z scanner. During the scan, the auto-Z signal at the detector 37 is measured (step S95). The operations of the X-Y scanner, the fast-Z scanner and the detector 37 are synchronized by the controller 40, so that there is a correspondence between the X-Y-Z position of the focal spot and the auto-Z signal. The peaks of the auto-Z signal are detected, and the corresponding X-Y-Z positions of the focal spot are determined based on this correspondence (step S96). The depth profile of the posterior corneal surface is reconstructed from these X-Y-Z positions, by adding the known offset distance $\delta$ (step S97) to the Z value. The data processing functions are performed by the controller.

Fig. 10A schematically illustrates an exemplary X-Y spiral scan pattern, and Fig. 10B schematically illustrates an exemplary fast-Z scan pattern. Thus, the laser focal spot will be moved up and down, along the spiral pattern. Note that the scanning effect of the resonant scanner is not illustrated in Fig. 10A. The frequency of the fast-Z oscillating scan and the speed of the X-Y spiral scan are controlled (by the controller 40) such that a desired number of Z scan oscillations occur along the spiral scan pattern. The initial depth (e.g. 550 $\mu$m below the anterior corneal surface) and the amplitude of the fast-Z scan (e.g. about 100 $\mu$m, so that the oscillation covers a depth range of 450 $\mu$m to 650 $\mu$m) are chosen based on an estimated approximate posterior corneal surface shape, so that within the X-Y area of the spiral pattern, each oscillation of the Z scan will cross the target depth (the depth at $\delta$ above the posterior corneal surface). Therefore, two peaks will be generated in the auto-Z signal in each oscillation of the fast-Z scan. This way, a desired number of profile data points are obtained, each point being the Y-Y-Z position corresponding to a peak in the auto-Z signal. In the schematic illustration of Fig. 10A, each small circle may correspond to a data point.

In one example, the fast-Z scan frequency is about 10 Hz and the linear speed of the X-Y scan is approximately 45 mm/s. Using these parameters, a depth mapping may be performed within 2 seconds resulting in 40 measured data points along a spiral of 90 mm in linear length. This is typically sufficient to reconstruct a depth profile of the posterior corneal surface. More generally, the fast-Z scan frequency may be from 10 to 40 Hz and the X-Y scan speed may be from 10 to 45 mm/s.

While a spiral pattern is shown in Fig. 10A, the X-Y scan pattern may alternatively be a raster pattern (formed of parallel lines) or a serpentine pattern, but the spiral pattern has the advantage of low acceleration. Other X-Y-Z scan patterns may be used, depending on surface geometry of the surface being mapped.

An advantage of the posterior corneal surface mapping methods described here is that the measurement beam is the same as the cutting beam except for the lower pulse energy; therefore, position calibration in X, Y and Z directions between the measurement tool and the cutting laser beam is always precisely maintained. This is not the case when OCT is used to perform posterior corneal surface mapping.

Once the posterior corneal surface profile is measured, the laser pulse energy is set to a treatment level (e.g., approximately 70 nJ, or more generally, between 40 and 90 nJ), and a deep lamellar incision may be made using the treatment laser beam, where the lamellar incision is parallel to and located at a predetermined distance from the posterior corneal surface (see Fig. 2).

Note that the lamellar incision is not always required to be parallel to the posterior corneal surface. In alternative embodiments, the distance between the lamellar incision and the posterior corneal surface may vary with the X-Y position. More generally, once the posterior corneal surface profile is measured, the lamellar incision may be formed so that it has any defined spatial relationship with the posterior corneal surface.

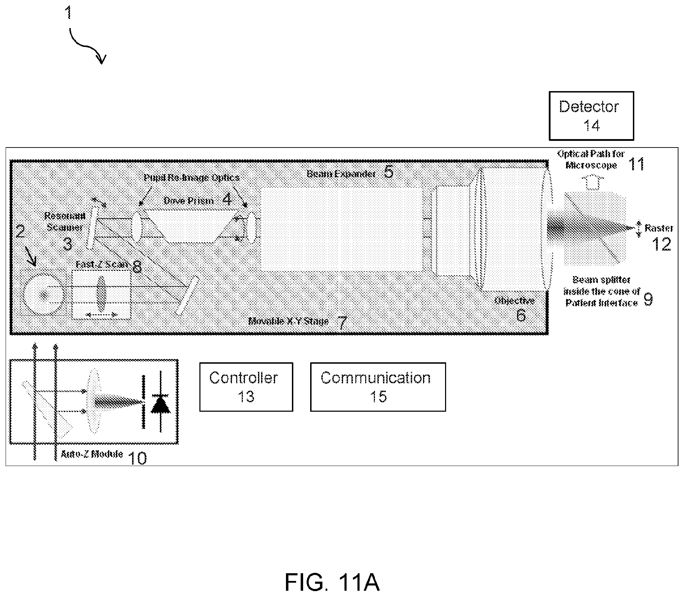

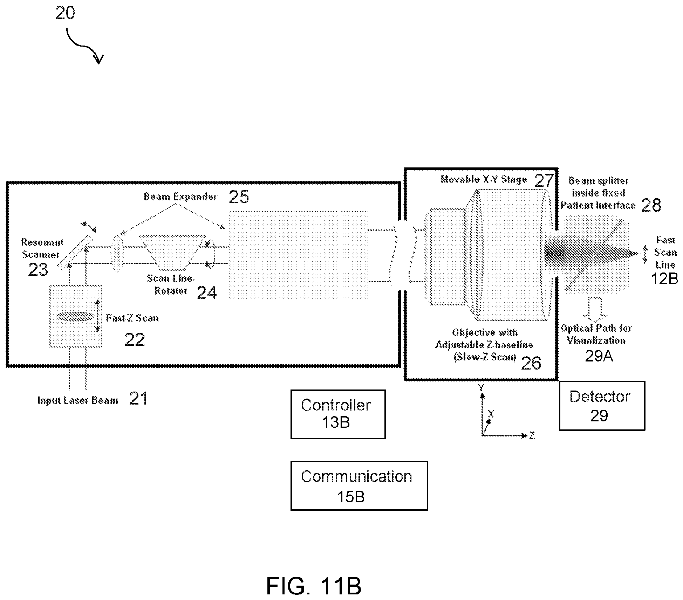

The laser source 31 that may be used to implement the above-described methods is described in more detail now with reference to Figs. 11A and 11B.

FIG. 11A shows an ophthalmic surgical laser system 1 suitable for making an incision in a target material such as a cornea of an eye. A laser 2, such as a femtosecond laser, provides a pulsed laser beam 2A which may be used in optical procedures to treat the eye. The system 1 further includes, but is not limited to, a high frequency scanner (such as a resonant scanner) 3 for scanning the pulsed laser beam to produce a scan line 12 of the pulsed laser beam, a scan line rotator 4 for rotating the scan line 12, a beam expander 5, an objective 6 for focusing the laser beam, an XY scan device 7 for deflecting or directing the laser beam on or within the target, a fast-Z scan device 8, a patient interface 9, an auto-Z device 10, a controller 13, and a communication module 15.

The resonant scanner 3 scans the pulsed laser beam at a high resonant frequency (e.g., thousands of Hz) to produces the scan line that extends in a lateral orientation (i.e. a direction perpendicular to the laser beam propagation direction Z) and having a desired length, for example, between 1 mm and 2 mm. The length of the scan line may be adjustable. The scan line rotator 4 may be implemented by a Dove prism, a Pechan prism, a set of mirrors, or the like, mounted on a rotating stage. By rotating the scan line rotator 4 around the Z axis, the lateral orientation of the scan line 12 is rotated, so that the scan line may be placed at any desired orientation in the XY plane (i.e., the lateral plane perpendicular to the laser beam propagation direction Z). The XY scan device 7 may be a movable XY scanning stage having the focusing objective 6 mounted thereon; the XY scan device 7 carries the objective 6 and moves it relative to the patient interface device 9, so as to move the center of the scan line 12 relative to the patient's eye in the XY directions. The fast-Z scan device 8 changes the depth (i.e. along the Z direction) of the laser focal spot location in the eye. Thus, the scan line rotator 4 modifies the lateral orientation of the scan line 12 while the moveable XY scanning stage 7 and the fast-Z scan device 8 move the center of the scan line in X, Y and Z directions. Because the scanning speed of the resonant scanner is typically much faster than the speed of the XY scanning stage and the fast-Z scan device, the scan line 12 may be referred to as a fast scan line, and the movement of the fast scan line in X, Y and Z directions may be referred to as a slow sweep.

The XY scanning stage 7 may be a motorized stage with two motors that drive its movements in the X and Y directions. Preferably, the XY scanning stage is a recoilless stage configured to reduce or eliminate mechanical vibration. The fast-Z scan device 8 may include a voice coil actuator that drives a lens in the Z direction. Movements of the lens lead to a focus depth change. The z-scan frequency may be between 50 Hz and 15,000 Hz.

The patient interface device 9 couples the patient's eye to the ophthalmic surgical laser system 1. The patient interface 9 may include a visualization beam splitter to reflect the light from the eye along an optical path 11 toward a video microscope or ocular microscope 14, to allow the eye to be imaged by an image detector of the microscope.

The auto Z module 10 may be either a confocal detector or a non-confocal detector.

The controller 13, which may be implemented by a processor executing suitable machine-readable program code and data stored in a non-volatile memory, is operably coupled to the various components of the system 1 including the laser 2, the fast-Z scan device 8, the resonant scanner 3, the scan line rotator 4, the XY scanning stage 7, the detector 14, and the communication module 15. The controller 13 is configured to direct these components of the system to output the focal spot of the pulsed laser beam in a desired pattern in the eye so as to modify the eye. The communication module 15 provides information to the operator of the laser system 1 at the system and/or remotely via wired or wireless data connection, and may include displays, user input devices such as keyboard, mouse, joystick, etc. The ophthalmic surgical laser system may additionally include an OCT (optical coherence tomography) device (not shown in FIG. 11A) which may be used to measure structures of the target (e.g. eye tissues).

FIG. 11B shows an ophthalmic surgical laser system 20 suitable for making an incision in a target material such as a cornea of an eye. The system 20 includes, but is not limited to, a laser source (not shown) that generates an input pulsed laser beam 21, a fast-Z scan device 22, a resonant scanner 23 for producing a scan line 12B of the pulsed laser beam 21, a scan line rotator 24 for rotating the lateral orientation of the scan line 12B, a beam expander 25, an objective with an adjustable focusing mechanism (slow-Z scanner) 26, a XY scanning stage 27 for deflecting or directing the pulsed laser beam 21 on or within the target, a patient interface 28 that may include a beam splitter, a controller 13B, an image detector 29 disposed on an optical path 29A defined by the beam splitter of the patient interface, and a communication module 15B. The slow-Z scanner 26 may be used to set the laser focal spot at a desired focal depth which may set the Z-baseline of the scan pattern.

One difference between the embodiment of FIG. 11B and that of FIG. 11A is that the XY scanning stage 7 in FIG. 11A carries both the objective 6 and other components including the fast-Z scan device 8, resonant scanner 3, scan line rotator 4, and beam expander 5, while the XY scanning stage 27 in FIG. 11B carries the objective 26 but not the other components mentioned above. Note that the in the system of FIG. 11A, the objective 6 may also be equipped with a slow-Z scanner (also represented by reference symbol 6).

The controller 13 or 13B may correspond to the controller 40 in Fig. 3; the auto Z module 10 may correspond to the detector 37 in Fig. 3; and the objective lens 6 may correspond to the objective lens 34 in Fig. 3.

Further details of ophthalmic surgical laser systems having the configurations shown in FIGS. 11A and 11B are described in commonly owned U.S. Pat. Appl. Nos. 14/970898, filed December 16, 2015, entitled "Compact Ultra-Short Pulsed Laser Eye Surgery Workstation," and 14/865396, filed September 25, 2015, entitled "Systems and Methods for Lenticular Laser Incision,".

In other embodiments, an ophthalmic surgical laser system may employ other types of scanners, such as two orthogonal scanning mirrors, for scanning the laser beam in the transverse (XY) directions. Many such systems are known and their details are not described here.

While specific reference is made to keratoplasty and ophthalmic procedures, the embodiments described herein can be used in other applications for improved tissue incisions with decreased irregularity of the incised tissue surface, as well as for more accurate tissue cutting with improved healing. Among other things, these additional applications include mapping anterior or posterior crystalline lens surface when performing incisions in the crystalline lens. The method may be used more generally as a tool for high precision corneal pachymetry. It may also be used to profile corneal defects such as internal scars within the cornea. More generally, the method is applicable as a measurement tool in any transparent material where a reflective interface is present.

It will be apparent to those skilled in the art that various modification and variations can be made in the posterior corneal surface mapping and deep lamellar corneal incision methods and related apparatus of the present invention without departing from the scope of the claims. Thus, it is intended that the present invention cover modifications and variations that come within the scope of the appended claims.

## Claims

1. An ophthalmic laser system (30) comprising:

   a pulsed laser (31) configured to generate a pulsed laser beam;
   an optical system including an objective lens (34) and one or more scanners, configured to focus the laser beam to a focus point (F) between the objective lens and a reflective interface in an eye of the patient and to scan the focus point (F) in three directions in the eye of a patient;
   a light intensity detector (37) disposed to detect a light intensity of a back-reflected laser beam from the eye that has passed through the objective lens (34),
   wherein the optical system and light intensity detector (37) are configured such that the forward propagating laser beam diverges after the focus point, is then reflected by the reflective interface, and wherein the reflected light is focused by the objective lens to an image point (D) at a finite distance (v) from the objective lens, and wherein the detector is located at the image point (D);
   a controller (40) electrically coupled to the pulsed laser (31), the optical system and the light intensity detector (37), wherein the controller (40) is configured to:

      control the pulsed laser (31) to generate a first laser beam having a first pulse energy lower than a threshold energy for photodisruption of corneal tissue;
      control the optical system to scan the focus point (F) of the first laser beam according to a first scan pattern, wherein the first scan pattern is at least partially located within a cornea of the eye and extends in a predetermined depth range in a depth direction which is parallel to an optical axis of the objective lens (34);

control the detector (37) to detect a light intensity signal of a back-reflected portion of the first laser beam while the focus point (F) of the first laser beam is scanned according to the first scan pattern;

based on a correspondence between the light intensity signal and the first scan pattern, determine a depth profile of a posterior corneal surface of the eye;

control the pulsed laser (31) to generate a second laser beam having a second pulse energy higher than the threshold energy; and

based on the determined depth profile of the posterior corneal surface, control the optical system to scan the focus point of the second laser beam within the cornea according to a second scan pattern, the second scan pattern having a defined spatial relationship with the determined posterior corneal surface profile, to form an incision in the cornea.

2. The ophthalmic laser system of claim 1, wherein the one or more scanners includes a Z scanner and an X-Y scanner, and wherein the controller is configured to scan the focus point of the first laser beam according to the first scan pattern by:

controlling the Z scanner to scan the focus point in the depth direction according to an oscillating function of time; and

simultaneously controlling the X-Y scanner to scan the focus point in a spiral pattern in a plane perpendicular to the depth direction.

3. The ophthalmic laser system of claim 1, wherein the one or more scanners includes a Z scanner, an X-Y scanner, and a resonance scanner, and wherein the controller is configured to scan the focus point of the first laser beam according to the first scan pattern by:

controlling the Z scanner to scan the focus point in the depth direction according to a oscillating function of time;

simultaneously controlling the X-Y scanner to scan the focus point in a spiral pattern in a plane perpendicular to the depth direction; and

simultaneously controlling the resonant scanner to scan the focus point into a scanline in the plane at a frequency higher than a frequency of the oscillating function of the Z scanner.

4. The ophthalmic laser system of claim 1, wherein the one or more scanners includes a Z scanner, an X-Y scanner, and a resonance scanner, and wherein the controller is configured to scan the focus point of the first laser beam according to the first scan pattern by:

controlling the Z scanner and the X-Y scanner to scan the focus point in three dimensions according to a pattern; and

simultaneously controlling the resonant scanner to scan the focus point into a scanline in a plane perpendicular to the depth direction.

5. The ophthalmic laser system of claim 1, wherein the depth range of the first scan pattern is between 450 $\mu$m and 650 $\mu$m.

6. The ophthalmic laser system of claim 1, wherein the controller is configured to determine the depth profile of the posterior corneal surface by:

detecting a plurality of peaks in the light intensity signal;

based on the correspondence between the light intensity signal and the first scan pattern, determining a plurality of spatial positions corresponding to the plurality of peaks in the light intensity signal; and

adding a predetermined offset distance to a depth value of each spatial position to obtain the depth profile of the posterior corneal surface, wherein the offset distance is a fixed value determined by a focal length of the objective lens and a length of an optical path from the light intensity detector to the objective lens.

7. The ophthalmic laser system of claim 1, wherein the eye is coupled to a patient interface device when the controller controls the optical system to scan the focus points of the first and second laser beams in the eye.

8. The ophthalmic laser system of claim 1, wherein the second scan pattern is parallel to and located at a predetermined distance from the posterior corneal surface profile.

9. The ophthalmic laser system of claim 1, wherein the optical system includes a beam splitter disposed to guide a portion of the laser beam from the pulsed laser to the objective lens and to guide a portion of the back-reflected laser beam, which has been reflected back by a reflective structure disposed in front of the objective lens and has passed through the objective lens, to the light intensity detector, wherein the optical path is free of any lens or pinhole between the objective lens and the light intensity detector.

10. The ophthalmic laser system of claim 1, wherein the light intensity detector has an effective detection area of 1 mm$^2$ or less.

11. The ophthalmic laser system of claim 1, wherein the objective lens is configured to focus the parallel light beam to the focus point having a size of 2 $\mu$m or less and has a numerical aperture of 0.4 or higher.


**Patentansprüche**

1. Ophthalmisches Lasersystem (30), umfassend:

einen gepulsten Laser (31), die konfiguriert ist, um einen gepulsten Laserstrahl zu erzeugen; ein optisches System, das eine Objektivlinse (34) und einen oder mehrere Scanner einschließt, das konfiguriert ist, um den Laserstrahl auf einen Fokuspunkt (F) zwischen der Objektivlinse und einer reflektierenden Grenzfläche in einem Auge des Patienten zu fokussieren und den Fokuspunkt (F) in drei Richtungen in dem Auge eines Patienten zu scannen; einen Lichtintensitätsdetektor (37), der angeordnet ist, um eine Lichtintensität eines rückreflektierten Laserstrahls vom Auge zu detektieren, der durch die Objektivlinse (34) hindurchgetreten ist, wobei das optische System und der Lichtintensitätsdetektor (37) derart konfiguriert sind, dass der sich vorwärts ausbreitende Laserstrahl nach dem Fokuspunkt divergiert, dann von der reflektierenden Grenzfläche reflektiert wird, und wobei das reflektierte Licht durch die Objektivlinse zu einem Bildpunkt (D) in einem endlichen Abstand (v) von der Objektivlinse fokussiert wird, und wobei sich der Detektor am Bildpunkt (D) befindet; eine Steuerung (40), die elektrisch an den gepulsten Laser (31), das optische System und den Lichtintensitätsdetektor (37) gekoppelt ist, wobei die Steuerung (40) konfiguriert ist, um:

das gepulste Lasersystem (31) zu steuern, um einen ersten Laserstrahl mit einer ersten Pulsenergie zu erzeugen, die niedriger ist als eine Schwellenenergie für eine Photodisruption von Hornhautgewebe; das optische System zu steuern, den Fokuspunkt (F) des ersten Laserstrahls gemäß einem ersten Abtastmuster abzutasten, wobei das erste Abtastmuster mindestens teilweise innerhalb einer Hornhaut des Auges angeordnet ist und sich in einem vorbestimmten Tiefenbereich in einer Tiefenrichtung erstreckt, die parallel zu einer optischen Achse der Objektivlinse (34) ist; das Detektorelement (37) zu steuern, um ein Lichtintensitätssignal eines rückreflektierten Anteils des ersten Laserstrahls zu detektieren, während der Fokuspunkt (F) des ersten Laserstrahls gemäß dem ersten Abtastmuster abgetastet wird; basierend auf einer Entsprechung zwischen dem Lichtintensitätssignal und dem ersten Abtastmuster, ein Tiefenprofil einer hinteren Hornhautoberfläche des Auges zu bestimmen; den gepulsten Laser (31) zu steuern, um einen zweiten Laserstrahl mit einer zweiten Pulsenergie zu erzeugen, die höher als die Schwellenenergie ist; und basierend auf dem bestimmten Tiefenprofil der hinteren Hornhautoberfläche, das optische System zu steuern, um den Fokuspunkt des zweiten Laserstrahls innerhalb der Hornhaut gemäß einem zweiten Abtastmuster abzutasten, wobei das zweite Abtastmuster eine definierte räumliche Beziehung zu dem bestimmten Profil der hinteren Hornhautoberfläche aufweist, um einen Einschnitt in der Hornhaut zu bilden.

2. Ophthalmisches Lasersystem nach Anspruch 1, wobei der eine oder die mehreren Scanner einen Z-Scanner und einen X-Y-Scanner einschließen, und wobei die Steuerung konfiguriert ist, um den Fokuspunkt des ersten Laserstrahls gemäß dem ersten Abtastmuster abzutasten durch:

Steuern des Z-Scanners zum Scannen des Fokuspunkts in der Tiefenrichtung gemäß einer oszillierenden Funktion der Zeit; und gleichzeitiges Steuern des X-Y-Scanners zum Abtasten des Fokuspunkts in einem spiralförmigen Muster in einer Ebene senkrecht zur Tiefenrichtung.

3. Ophthalmisches Lasersystem nach Anspruch 1, wobei der eine oder die mehreren Scanner einen Z-Scanner, einen X-Y-Scanner und einen Resonanzscanner einschließen, und wobei die Steuerung konfiguriert ist, um den Fokuspunkt des ersten Laserstrahls gemäß dem ersten Abtastmuster abzutasten durch:

Steuern des Z-Scanners zum Scannen des Fokuspunkts in der Tiefenrichtung gemäß einer oszillierenden Funktion der Zeit; gleichzeitiges Steuern des X-Y-Scanners zum Scannen des Fokuspunkts in einem spiralförmigen Muster in einer Ebene senkrecht zur Tiefenrichtung; und

gleichzeitiges Steuern des Resonanzscanners zum Scannen des Fokuspunkts in eine Scanlinie in der Ebene bei einer Frequenz, die höher ist als eine Frequenz der oszillierenden Funktion des Z-Scanners.

4. Ophthalmisches Lasersystem nach Anspruch 1, wobei der eine oder die mehreren Scanner einen Z-Scanner, einen X-Y-Scanner und einen Resonanzscanner einschließen, und wobei die Steuerung konfiguriert ist, um den Fokuspunkt des ersten Laserstrahls gemäß dem ersten Abtastmuster abzutasten durch:

Steuern des Z-Scanners und des X-Y-Scanners zum Abtasten des Fokuspunkts in drei Dimensionen gemäß einem Muster; und gleichzeitiges Steuern des Resonanzscanners zum Scannen des Fokuspunkts in eine Scanlinie in einer Ebene senkrecht zur Tiefenrichtung.

5. Ophthalmisches Lasersystem nach Anspruch 1, wobei der Tiefenbereich des ersten Abtastmusters zwischen 450 μm und 650 μm liegt.

6. Ophthalmisches Lasersystem nach Anspruch 1, wobei die Steuerung konfiguriert ist, um das Tiefenprofil der hinteren Hornhautoberfläche zu bestimmen durch:

Detektieren einer Vielzahl von Spitzen in dem Lichtintensitätssignal; basierend auf der Entsprechung zwischen dem Lichtintensitätssignal und dem ersten Abtastmuster, Bestimmen einer Vielzahl von räumlichen Positionen, die der Vielzahl von Spitzen in dem Lichtintensitätssignal entsprechen; und Hinzufügen eines vorbestimmten Versatzabstands zu einem Tiefenwert jeder räumlichen Position, um das Tiefenprofil der hinteren Hornhautoberfläche zu erhalten, wobei der Versatzabstand ein fester Wert ist, der durch eine Brennweite der Objektivlinse und eine Länge eines optischen Pfads von dem Lichtintensitätsdetektor zu der Objektivlinse bestimmt wird.

7. Ophthalmisches Lasersystem nach Anspruch 1, wobei das Auge an eine Patientenschnittstellenvorrichtung gekoppelt ist, wenn die Steuerung das optische System steuert, um die Fokuspunkte des ersten und zweiten Laserstrahls in dem Auge zu scannen.

8. Ophthalmisches Lasersystem nach Anspruch 1, wobei das zweite Abtastmuster parallel zu dem Profil der hinteren Hornhautoberfläche ist und in einem vorbestimmten Abstand davon angeordnet ist.

9. Ophthalmisches Lasersystem nach Anspruch 1, wobei das optische System einen Strahlteiler einschließt, der angeordnet ist, um einen Anteil des Laserstrahls von dem gepulsten Laser zu der Objektivlinse zu führen und um einen Anteil des rückreflektierten Laserstrahls, der von einer reflektierenden Struktur, die vor der Objektivlinse angeordnet ist, zurückreflektiert wurde und durch die Objektivlinse hindurchgetreten ist, zu dem Lichtintensitätsdetektor zu führen, wobei der optische Pfad frei von jeder Linse oder Lochblende zwischen der Objektivlinse und dem Lichtintensitätsdetektor ist.

10. Ophthalmisches Lasersystem nach Anspruch 1, wobei der Lichtintensitätsdetektor eine effektive Detektionsfläche von 1 mm$^2$ oder weniger aufweist.

11. Ophthalmisches Lasersystem nach Anspruch 1, wobei die Objektivlinse konfiguriert ist, um den parallelen Lichtstrahl auf den Fokuspunkt mit einer Größe von 2 μm oder weniger zu fokussieren und eine numerische Apertur von 0,4 oder höher aufweist.

**Revendications**

1. Système laser ophtalmique (30) comprenant :

un laser pulsé (31) configuré pour générer un faisceau laser pulsé ; un système optique comportant une lentille d'objectif (34) et un ou plusieurs dispositifs de balayage, configurés pour focaliser le faisceau laser sur un point focal (F) entre la lentille d'objectif et une interface réfléchissante dans un œil du patient et pour balayer le point focal (F) dans trois directions dans l'œil d'un patient ; un détecteur d'intensité de lumière (37) disposé pour détecter une intensité de lumière d'un faisceau laser rétroréfléchi par l'œil, qui a traversé la lentille d'objectif (34), dans lequel le système optique et le détecteur d'intensité de lumière (37) sont configurés de sorte que le faisceau laser se propageant vers l'avant diverge après le point focal, est ensuite réfléchi par l'interface réfléchissante, et dans lequel la lumière réfléchie est focalisée par la lentille d'objectif sur un point d'image (D) à une distance finie (v) de la lentille d'objectif, et dans lequel le détecteur est localisé au niveau du point d'image (D) ; une unité de commande (40) couplée électriquement au laser pulsé (31), au système optique et au détecteur d'intensité de lumière (37), dans lequel l'unité de commande (40) est configurée pour :

commander le laser pulsé (31) pour générer un premier faisceau laser ayant une première énergie d'impulsion inférieure à une énergie seuil pour photodisruption du tissu cornéen ;

commander le système optique pour balayer le point focal (F) du premier faisceau laser selon un premier motif de balayage, dans lequel le premier motif de balayage est au moins partiellement localisé au sein d'une cornée de l'œil et s'étend dans une plage de profondeur prédéterminée dans une direction de profondeur qui est parallèle à un axe optique de la lentille d'objectif (34) ;

commander le détecteur (37) pour détecter un signal d'intensité de lumière d'une partie rétroréfléchie du premier faisceau laser alors que le point focal (F) du premier faisceau laser est balayé selon le premier motif de balayage ;

en fonction d'une correspondance entre le signal d'intensité de lumière et le premier motif de balayage, déterminer un profil de profondeur d'une surface cornéenne postérieure de l'œil ;

commander le laser pulsé (31) pour générer un second faisceau laser ayant une seconde énergie d'impulsion supérieure à l'énergie seuil ; et

en fonction du profil de profondeur déterminé de la surface cornéenne postérieure, commander le système optique pour balayer le point focal du second faisceau laser au sein de la cornée selon un second motif de balayage, le second motif de balayage ayant une relation spatiale définie avec le profil de surface cornéenne postérieure déterminé, pour former une incision dans la cornée.

2. Système laser ophtalmique selon la revendication 1, dans lequel le ou les dispositifs de balayage comportent un dispositif de balayage Z et un dispositif de balayage X-Y, et dans lequel l'unité de commande est configurée pour balayer le point focal du premier faisceau laser selon le premier motif de balayage par :

la commande du dispositif de balayage Z pour balayer le point focal dans la direction de profondeur selon une fonction de temps oscillante ; et

la commande simultanée du dispositif de balayage X-Y pour balayer le point focal dans un motif en spirale dans un plan perpendiculaire à la direction de profondeur.

3. Système laser ophtalmique selon la revendication 1, dans lequel le ou les dispositifs de balayage comportent un dispositif de balayage Z, un dispositif de balayage X-Y et un dispositif de balayage à résonance, et dans lequel l'unité de commande est configurée pour balayer le point focal du premier faisceau laser selon le premier motif de balayage par :

la commande du dispositif de balayage Z pour balayer le point focal dans la direction de profondeur selon une fonction de temps oscillante ;

la commande simultanée du dispositif de balayage X-Y pour balayer le point focal dans un motif en spirale dans un plan perpendiculaire à la direction de profondeur ; et

la commande simultanée du scanner à résonance pour balayer le point focal dans une ligne de balayage dans le plan à une fréquence supérieure à une fréquence de la fonction oscillante du dispositif de balayage Z.

4. Système laser ophtalmique selon la revendication 1, dans lequel le ou les dispositifs de balayage comportent un dispositif de balayage Z, un dispositif de balayage X-Y et un dispositif de balayage à résonance, et dans lequel l'unité de commande est configurée pour balayer le point focal du premier faisceau laser selon le premier motif de balayage par :

la commande du dispositif de balayage Z et du dispositif de balayage X-Y pour balayer le point focal dans trois dimensions selon un motif ; et

la commande simultanée du scanner à résonance pour balayer le point focal dans une ligne de balayage dans un plan perpendiculaire à la direction de profondeur.

5. Système laser ophtalmique selon la revendication 1, dans lequel la plage de profondeur du premier motif de balayage est entre 450 μm et 650 μm.

6. Système laser ophtalmique selon la revendication 1, dans lequel l'unité de commande est configurée pour déterminer le profil de profondeur de la surface cornéenne postérieure par :

la détection d'une pluralité de pics dans le signal d'intensité de lumière ;

en fonction de la correspondance entre le signal d'intensité de lumière et le premier motif de balayage, la détermination d'une pluralité de positions spatiales correspondant à la pluralité de pics dans le signal d'intensité de lumière ; et

l'ajout d'une distance de décalage prédéterminée à une valeur de profondeur de chaque position spatiale pour obtenir le profil de profondeur de la surface cornéenne postérieure, dans lequel la distance de décalage est une valeur fixe déterminée par une longueur focale de la lentille d'objectif et une longueur d'un trajet optique allant du détecteur d'intensité de lumière à la lentille d'objectif.

7. Système laser ophtalmique selon la revendication 1, dans lequel l'œil est accouplé à un dispositif d'interface patient lorsque l'unité de commande effectue la commande du système optique pour balayer les points focaux des premier et second faisceaux laser dans l'œil.

8. Système laser ophtalmique selon la revendication 1, dans lequel le second motif de balayage est parallèle à, et localisé à, une distance prédéterminée du profil de surface cornéenne postérieure.

9. Système laser ophtalmique selon la revendication 1, dans lequel le système optique comporte un diviseur de faisceau disposé pour guider une partie du faisceau laser provenant du laser pulsé vers la lentille d'objectif et pour guider une partie du faisceau laser rétroréfléchi, qui a été renvoyé par réflexion par une structure réfléchissante disposée devant la lentille d'objectif et a traversé la lentille d'objectif, vers le détecteur d'intensité de lumière, dans lequel le trajet optique est exempt de lentille ou trou d'épingle quelconque entre la lentille d'objectif et le détecteur d'intensité de lumière.

10. Système laser ophtalmique selon la revendication 1, dans lequel le détecteur d'intensité de lumière a une zone de détection efficace de 1 mm$^2$ ou moins.

11. Système laser ophtalmique selon la revendication 1, dans lequel la lentille d'objectif est configurée pour focaliser le faisceau de lumière parallèle sur le point focal ayant une taille de 2 $\mu$m ou moins et a une ouverture numérique de 0,4 ou plus élevée.

Fig. 1 (Prior art)

Fig. 2

Fig. 3

Fig. 4A

Fig. 4B

S51: Dock patient's eye to patient interface

↓

S52: Measure depth profile of posterior corneal surface using lower-energy detecting laser beam

↓

S53: Using higher-energy treatment laser beam, form deep lamellar incision parallel to and at a predetermined distance from posterior corneal surface

Fig. 5

S61: Reduce laser pulse energy to lower level

↓

S62: At each X-Y position, focus laser beam to a depth approximately δ above the posterior corneal surface and scan laser focal spot up and/or down

↓

S63: Measure auto-Z signal

↓

S64: Detect peaks of auto-Z signal

↓

S65: Calculate depth of posterior corneal surface based on detected peak of auto-Z signal and δ

↓

S66: Move to another X-Y position; repeat steps S62-S65

Fig. 6

Fig. 7

Fig. 8

S91: Reduce laser pulse energy to lower level

↓

S92: Set resonant scanner to scanline length of about 100 μm

↓

S93: Operate slow-Z scanner to focus laser beam near center of cornea at a desired depth (e.g. 550 μm)

↓

S94: Execute scan: resonant scanner scans laser focal spot in X-Y plane along a scanline; fast-Z scanner scans depth of focal spot as sinusoidal function of time; X-Y scanner scans focal spot in X-Y plane along spiral pattern

↓

S95: Measure auto-Z signal at the detector

↓

S96: Detect peaks of auto-Z signal and determine corresponding X-Y-Z positions of laser focal spot

↓

S97: Construct posterior corneal surface profile from peak X-Y-Z positions and δ

Fig. 9

Fig. 10A

Fig. 10B

FIG. 11A

20

Beam Expander 25

Resonant Scanner 23

Scan-Line-Rotator 24

Fast Z Scan 22

Input Laser Beam 21

Movable X-Y Stage 27

Beam splitter inside fixed Patient Interface 28

Fast Scan Line 12B

Optical Path for Visualization 29A

Objective with Adjustable Z-baseline (Slow-Z Scan) 26

Controller 13B

Communication 15B

Detector 29

FIG. 11B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070027438
- US 10080684 B
- US 20070027438 A
- US 20150374549 A
- US 20150272782 A
- US 202006422 A1
- US 11250718
- US 16112507 B
- US 97089815
- US 14865396 B